# EUROPEAN PATENT SPECIFICATION

(11) **EP 1 046 716 B1**
(45) Date of publication and mention of the grant of the patent: **24.03.2010**
(21) Application number: 98938983.8
(22) Date of filing: 25.08.1998
(51) Int. Cl.: C12Q 1/60

(54) **METHODS FOR QUANTITATING HIGH-DENSITY LIPOPROTEIN CHOLESTEROL**
VERFAHREN ZUR QUANTIFIZIERUNG VON LIPOPROTEIN-CHOLESTEROL HOHER DICHTE
METHODES DE QUANTIFICATION DE CHOLESTEROL DES LIPOPROTEINES DE HAUTE DENSITE

(30) Priority: 27.08.1997 JP 24482197
(43) Date of publication of application: 25.10.2000
(60) Divisional application: 09004707.7
(73) Proprietor: Sekisui Medical Co., Ltd., Tokyo 103-0027 (JP)
(72) Inventor: NAKANISHI, Kazuo-Daiichi Pure Chemicals Co., Ltd., Ibaraki-ken 301-0852 (JP); NAKAMURA, Mitsuhiro-Daiichi Pure Chemicals Co., Lt, Ibaraki-ken 301-0852 (JP); HINO, Koichi-Daiichi Pure Chemicals Co., Ltd., Ibaraki-ken 301-0852 (JP); MANABE, Mitsuhisa-Daiichi Pure Chemicals Co., Ltd., Ibaraki-ken 301-0852 (JP)
(74) Representative: Teasdale, Nicola Joanne
(86) International application number: PCT/JP1998/003771
(87) International publication number: WO 1999/010526

(56) References cited:
- JP-A- 7 301 636
- JP-A- 9 000 299
- JP-A- 9 313 200
- JP-A- 63 126 498
- US-A- 4 851 335
- US-A- 4 892 815
- DATABASE CHEMABS [Online] CHEMICAL ABSTRACTS SERVICE, COLUMBUS, OHIO, US; TAMURA, MUTSUHIKO ET AL: "Multilayer test element comprising fractionation agent for determination of high density lipoproteins" XP002285696 retrieved from STN Database accession no. 114:118086 & JP 02 210265 A2 (KONICA CO., JAPAN) 21 August 1990 (1990-08-21)

## Description

### Technical Field

This invention relates to a method which with a small sample quantity, permits efficient quantitation of cholesterol in high-density lipoprotein (HDL) only separately from cholesterol in lipoproteins other than HDL by simple procedures without needing procedures such as centrifugation.

### Background Art

Lipids such as cholesterol are complexed with apoproteins in serum to form lipoproteins. Depending on differences in physical properties, lipoproteins are classified into chylomicron, very low-density lipoprotein (VLDL), low-density lipoprotein (LDL), high-density lipoprotein (HDL), and so on. Among these lipoproteins, LDL is known to be one of causative substances which induce arteriosclerosis, while HDL is known to show anti-arteriosclerotic activity.

Epidemiologically, the level of cholesterol in HDL is known to show an inverse correlation with the frequency of onset of arteriosclerotic disease. These days, measurements of cholesterol in HDL are widely conducted for the prevention or diagnosis of ischemic heart diseases. As methods known for the measurement of cholesterol in HDL, there are, for example, a method in which HDL is separated from other lipoproteins by ultracentrifugal separation and is then subjected to a cholesterol measurement; and another method in which subsequent to separation of HDL from other lipoproteins by electrophoresis, its lipid is stained, and the intensity of a developed color is measured. These methods, however, have not been used routinely, because they involve one or more problems in that procedures are intricate and many samples cannot be handled.

A method for the measurement of cholesterol in HDL, which is generally and widely used at present in the field of clinical tests, is the precipitation method in which a precipitation reagent is added to a sample to agglutinate lipoproteins other than HDL, the resulting agglutinate is removed by centrifugation, and cholesterol in supernatant which contains only HDL so isolated is then measured. This method is simpler compared with ultracentrifugation or electrophoresis, but due to the inclusion of the procedures to add the precipitation reagent and to perform the separation, requires each sample in a relatively large quantity, involves a potential problem of producing an analytical error, and does not make it possible to fully automate the entire analysis steps.

On the other hand, methods have been studied for the enzymatic fractional quantitation of cholesterol in HDL. Known methods include, for example, to conduct an enzymatic reaction in the presence of a bile salt and a nonionic surfactant (JP 63-126498 A). This method makes use of the fact that an enzymatic reaction proceeds in proportion to an LDL concentration in an initial stage of the reaction and then in proportion to the concentration of cholesterol in HDL. A problem however exists in accuracy because the reaction with the cholesterol in HDL and the reaction with cholesterol in other lipoproteins cannot be fully distinguished.

Also included in the known methods is to have lipoproteins other than HDL agglutinated in advance, to cause cholesterol in HDL alone to react enzymatically, and to inactivate the enzyme and at the same time, to redissolve the agglutinate, followed by the measurement of an absorbance (JP 6-242110 A). This method, how-, ever, requires at least three procedures to add reagents so that it can be applied only to particular automatic analyzers, leading to a problem in wide applicability. Further, this method is not satisfactory from the standpoint of damages to analytical equipments and disposal of the reagents because of the use of a salt at a high concentration upon redissolution of an agglutinate.

Furthermore, Japanese Patent No. 2,600,065 discloses a method which makes combined use of a precipitation reagent, which is used in general precipitation methods and causes precipitation of lipoproteins other than HDL, and an ordinary cholesterol measuring reagent to measure the unprecipitated cholesterol in HDL. It is however impossible to measure cholesterol in HDL no matter whichever Example in this patent is followed. Therefore, this patent is considered to be such a patent as failing to include an essential element of its invention by error, and cannot serve as prior art.

### Disclosure of the Invention

Concerning the measurement of cholesterol in serum lipoproteins, the present inventors have proceeded with an investigation in various ways. In the course of the investigation, it was found that, when a reaction is conducted between serum and a cholesterol-quantitating enzyme reagent in the presence of a specific surfactant capable of dissolving lipoproteins, only HDL cholesterol reacts first, cholesterol in VLDL reacts next after the reaction of HDL, and with a substantial delay, cholesterol in LDL reacts finally.

As a result of further research, it has also been found that the HDL cholesterol alone can be measured by selecting a measurement point as desired such that the first reaction which relies upon the concentration of the HDL cholesterol can be measured, that the reaction relying upon the concentration of the HDL cholesterol alone can be maintained over an extended time by making a substance, which inhibits the reaction between the cholesterol in serum lipoproteins and the cholesterol-quantitating reagent, exist in the system, and that these methods can also be applied to automatic analyzers, leading to the completion of the present disclosure.

The present disclosure therefore provides a method for the quantitation of cholesterol in high-density lipoprotein, which comprises adding a surfactant, which is selected from polyoxyethylene alkylene phenyl ethers and polyoxyethylene alkylene tribenzylphenyl ethers, and a cholesterol-quantitating enzyme reagent to serum; and then measuring a reacted quantity of the cholesterol in the high-density lipoprotein in a time in which the cholesterol in the high-density lipoprotein preferentially reacts with the cholesterol-quantitating enzyme reagent.

The present disclosure also provides a method for the quantitation of HDL cholesterol as described above, wherein a substance having effect to inhibit a reaction between cholesterol in the serum lipoproteins and the cholesterol-quantitating enzyme reagent is added further.

The present disclosure also provides a quantitation enzyme and a reagent kit, which make it possible to advantageously practice each of the above-described methods.

### Brief Description of the Drawings

FIG. 1 is a diagram showing a correlation between analysis data obtained by the quantitation method disclosed herein and those obtained by the precipitation method in Example 1; and
FIG. 2 is a diagram illustrating a correlation between analysis data obtained by the quantitation method disclosed herein and those obtained by the precipitation method in Example 2.

### Best Modes for Carrying Out the Invention

The surfactant (hereinafter called the "lipoprotein dissolving agent") for use in the quantitation method disclosed herein, which is selected from polyoxyethylene alkylene phenyl ethers and polyoxyethylene alkylene tribenzylphenyl ethers, is a surfactant having effect to dissolve lipoproteins. These surfactants can include "Emulgen A-60" (product of Kao Corporation) as a commercially-available example of the former and "Emulgen B66" (product of Kao Corporation) as a commercially-available example of the latter.

These surfactants can be used either singly or in combination. The amount of the surfactant to be used varies depending on the kind of the surfactant, and no particular limitation is imposed thereon. It can be empirically determined for each analyzer, to which the reagent is applied, such that the sensitivity of the analyzer is set to permit detection of HDL cholesterol within a desired measurement time. In general, it is preferred to use the surfactant at a concentration of from 0.01 to 5 wt.%.

The quantitation method according to the present disclosure may preferably be conducted in the presence of a substance (hereinafter called the "reaction inhibitor") having effect to inhibit the reaction between cholesterol in serum lipoproteins and the cholesterol-quantitating enzyme reagent. Compared with a case where no reaction inhibitor exists, the presence of the reaction inhibitor makes it possible to retain for a longer time a reaction which relies only upon the concentration of HDL cholesterol.

Illustrative of the reaction inhibitor for use in the herein disclosed quantitation method are substances which exhibit binding affinity to lipoproteins and also surfactants which do not dissolve lipoproteins. They can be used either singly or in combination.

Examples of the substances (hereinafter called the "binding substances") which exhibit binding affinity to lipoproteins can include combinations of polyanions and substances capable of forming divalent metal salts. They can also be such substances as being complexed with HDL to form precipitates. Specific examples of the polyanions can include dextran sulfate, phosphotungstic acid and heparin, while examples of the substances capable of forming divalent metal salts can include the chlorides of divalent metals, such as MgCl₂, CaCl₂, MnCl₂ and NiCl₂, and their hydrates. These binding substances can be used either singly or in combination. The amount of the binding substance to be used varies depending upon its type, and no particular limitation is imposed thereon. It is, however, desired to use them in ranges such that as final concentrations in the reaction, the polyanion amounts to 0.002 to 10 wt.% and the substance capable of forming a divalent metal salt amounts to 0.01 to 5 wt.%, respectively.

Further, the surfactant (hereinafter called the "lipoprotein non-dissolving agent") which does not dissolve lipoproteins can be, for example, a surfactant selected from polyoxyethlene alkyl ethers, polyoxyethylene alkylphenyl ethers, polyoxyethylene-polyoxypropylene condensate, polyoxyethylene alkyl ether sulfates, and alkylbenzenesulfonate salts.

Among these, preferred are polyoxyethylene cetyl ether [as a commercial product, "Emulgen 220" (product of Kao Corporation)] as a polyoxyethylene alkyl ether; polyoxyethylene nonylphenyl ether [as a commercial product, "Emulgen 913" (product of Kao Corporation)] as a polyoxy ethylene alkylphenyl ether; "Pluronic F-88" (product of Asahi Denka K.K.) as a polyoxyethylene-polyoxypropylene condensate; polyoxyethylene lauryl ether sulfate sodium salt [as a commercial product, "EMAL 20C" (product of Kao Corporation)]; and sodium dodecylbenzenesulfonate as an alkylbenzenesulfonate.

These lipoprotein non-dissolving agents can be used either singly or in combination. No particular limitation is imposed on its amount to be used, but it is desired to use it in such a range that, when mixed with a sample, its concentration amounts to 0.01 to 5 wt.%, especially 0.05 to 1 wt.%.

Upon addition of the lipoprotein dissolving agent and the cholesterol-quantitating enzyme reagent (hereinafter called the "cholesterol reagent") to serum as a sample, they may be added separately or they may be added concurrently as a mixture. On the other hand, the reaction inhibitor can be used by adding it to one of the lipoprotein dissolving agent and the cholesterol reagent or to a mixture thereof. When plural reaction inhibitors are used, they can be mixed together and can be used in a similar manner as in the case of the use of the single reaction inhibitor. As an alternative, they can be added separately to the lipoprotein-dissolving agent and the cholesterol reagent, respectively.

Examples of the cholesterol reagent useful for the quantitation of cholesterol in the present disclosure can include known cholesterol reagents such as a combination of cholesterol esterase and cholesterol oxidase and a combination of cholesterol esterase and cholesterol dehydrogenase. Of these, the combination of cholesterol esterase and cholesterol oxidase is preferred.

No particular limitation is imposed on a method which is used to finally measure the reacted quantity of cholesterol subsequent to the addition of these cholesterol reagents. Illustrative are absorbance spectroscopy which is performed by further combining a peroxidase with a chromogen, and a method in which a coenzyme or hydrogen peroxide is directly detected.

In the method of the present disclosure it is necessary to detect the reaction between HDL cholesterol and the cholesterol-quantitating enzyme reagent in the time in which HDL cholesterol preferentially reacts with the cholesterol-quantitating enzyme reagent. Illustrative methods usable for the above detection can include a method in which a reaction which proceeds subsequent to the mixing of the lipoprotein dissolving agent, the cholesterol reagent and the sample is dynamically monitored; and a method (two-point method) in which the reaction of HDL cholesterol is measured by the reaction end-point method and the result of the measurement is corrected by a blank value. When there is a need to monitor the reaction of HDL for an extended time, the reaction which relies upon the concentration of HDL cholesterol alone can be prolonged by adding a reaction inhibitor and hence delaying the cholesterol detection reaction.

The followings are examples of a reagent or reagent kit usable for the advantageous practice of the method of the present disclosure:
(1) Reagents or reagent kits including the following two ingredients (a) and (b):
   (a) Lipoprotein dissolving agent, and
   (b) Cholesterol reagent.
(2) Reagents or reagent kits including the following ingredients (a) to (c):
   (a) Lipoprotein dissolving agent,
   (b) Reaction inhibitor (binding substance, lipoprotein non-dissolving agent), and
   (c) Cholesterol reagent.

These reagents or reagent kits can be combined with the above-described lipoprotein dissolving agents, cholesterol reagents (cholesterol esterase, cholesterol oxidase, cholesterol esterase, cholesterol dehydrogenase, etc.), reaction inhibitors (binding substances, lipoprotein non-dissolving agents), peroxidase, chromogens, coenzymes, appropriate pH buffers, antioxidants, carriers, and/or the like. Illustrative forms of such reagents or reagent kits can include, in addition to liquid forms, those obtained by lyophilizing such liquid forms.

### Examples

This invention will next be described further by Examples. It should however be borne in mind that the present invention is by no means limited to or by the Examples.

### Example 1

With respect to each of 50 serum samples containing lipoproteins, the cholesterol in HDL was quantitated by the quantitation method of the present disclosure and the conventional precipitation method, and the measurement values were compared.

According to the quantitation method disclosed herein, 100 mM MES buffer (First Reagent; pH 6.5) (300 µL) was added to each serum sample (3 µL), and about 5 minutes later, a cholesterol quantitation reagent (Second Reagent) (100 µL) - which was composed of 1% "Emulgen B-66", 1 U/mL cholesterol esterase, 1 U/mL cholesterol oxidase, 5 U/mL peroxidase, and 100 mM MES buffer (pH 6.5) containing 0.04% disulfobutylmetatoliudine and 0.004% 4-aminoantipyrine - was added. Shortly before the addition of Second Reagent and five minutes after its addition, the absorbance was measured at 600 nm (subsidiary wavelength: 700 nm). From a difference in absorbance, the concentration of HDL cholesterol in the serum sample was determined (2-point method). As a calibration substance, a control serum sample with a known HDL cholesterol concentration was used. The above procedures were conducted using an automatic analyser "Hitachi Model 7150".

To quantitate the cholesterol in HDL by the precipitation method, on the other hand, an aqueous solution (200 µL) which contained 0.3% of dextran sulfate and 2% magnesium chloride was mixed with the sample (200 µL), followed by centrifugation at 3,000 rpm for 10 minutes. The supernatant (50 µL) was collected, followed by the mixing with a cholesterol quantitation reagent (3 mL) composed of 1% Triton X-100, 1 U/mL cholesterol esterase, 1 U/mL cholesterol oxidase, 5 U/mL peroxidase, and 100 nM MES buffer (pH 6.5) containing 0.04% disulfobutylmetatoluidine and 0.004% 4-aminoantipyrine. After the resulting mixture was incubated at 37°C for 10 minutes, its absorbance at 600 nm was measured to determine the concentration of the cholesterol in HDL.

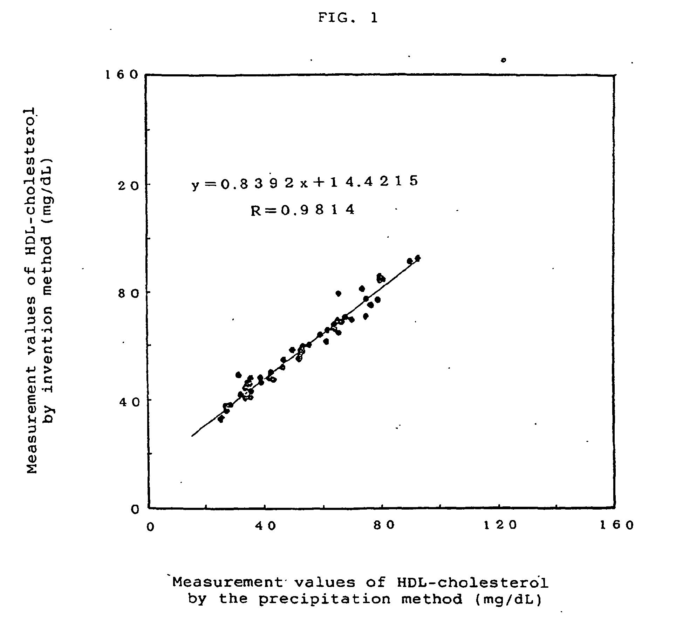

A correlation diagram of the results obtained by the quantitation method and the precipitation method are shown in FIG.1. The quantitation method, despite its simple procedures, showed an extremely good correlation with the conventional precipitation method.

### Example 2

With respect to each of 50 serum samples, the cholesterol in HDL was quantitated using the same reagents and the same procedures as in Example 1 except that 1% "Emulgen A-60" was added instead of "Emulgen B-66" in Second Reagent.

Described specifically, First Reagent (300 µL) was added to the sample (3 µL), and about five minutes later, Second Reagent (10.0 µL) was added. Variations in absorbance at 546 nm (subsidiary wavelength: 660 nm) from the 12th second to the 24th second subsequent to the addition of Second Reagent were measured, and the concentration of HDL cholesterol in the serum sample was determined. As calibration substances, two control serum samples with known concentrations (low concentration and high concentration) were used. The above procedures were conducted using the automatic analyzer "Hitachi Model 7150".

With respect to the same sample, the cholesterol in HDL was quantitated by the same precipitation method in a similar manner as in Example 1. Those measurement values were compared. The results are shown in FIG.

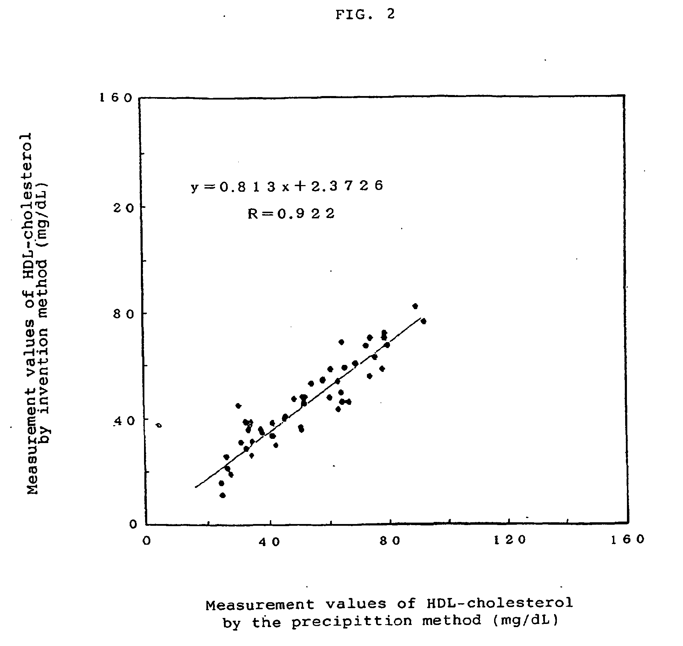

From the results of FIG. 2, the quantitation method disclosed herein, despite its simple procedures, showed a good correlation with the conventional precipitation method.

### Example 3

Using the same Second Reagent as that employed in Example 2 and the below-described First Reagent, the cholesterol in HDL in each of 50 serum samples, which contained lipoproteins, was quantitated by the quantitation method disclosed herein and the conventional quantitation method, and the measurement values were compared. Incidentally, First Reagent A (same as that in Example 2) was added as a control.

### [First Reagent]

First Reagent A:
   100 mM MES buffer (pH 6.5).
First Reagent B:
   0.2% aqueous solution of "Pluronic F-88" (product of Asahi Denka K.K.).
First Reagent C:
   An aqueous solution containing 0.2% sodium phosphotungstate and 100 mM magnesium chloride (pH 6.4).
First Reagent D:
   An aqueous solution containing 0.2% "Pluronic F-88", 0.2% sodium phosphotungstate and 100 mM magnesium chloride (pH 6.4).

To confirm effects of the addition of the reaction inhibitors, the absorbance was measured using different measurement times, that is, from the 12th second to the 24th second, from the 12th second to the 168th second, and from the 12th second to the 312th second, all after the addition of Second Reagent, by using "Hitachi Model 7150".

On the other hand, measurements of HDL by the precipitation method were conducted in a similar manner as in Example 1, and an investigation was made for a correlation with the quantitation method under the same conditions. Correlation coefficients are shown in Table 1.

**Table 1**

| | Measurement time | | |
|---|---|---|---|
| | 12-24 | 12-168 | 12-312 |
| | seconds | seconds | seconds |
| First Reagent A | 0.922 | 0.488 | 0.336 |
| First Reagent B | 0.739 | 0.900 | 0.846 |
| First Reagent C | 0.543 | 0.946 | 0.747 |
| First Reagent D | 0.045 | 0.972 | 0.988 |

As is appreciated from the results, the First Reagent which did not contain any reaction inhibitor showed good results only in the measurement of the absorbance in the initial stage of the reaction. On the other hand, according to the results of the measurements in which the reagents containing one or more reaction inhibitors, i.e., the 0.2% aqueous solution of "Pluronic F-88" (product of Asahi Denka K.K.), the aqueous solution containing 0.2% sodium phosphotungstate and 100 mM magnesium chloride (pH 6.4) and the aqueous solution containing 0.2% "Pluronic F-88", 0.2% sodium phosphotungstate and 100 mM magnesium chloride (pH 6.4) were used as First Reagents, respectively, all the results showed good correlations (the closer to 1, the higher the correlation) in the measurements over the extended times. It has therefore been confirmed that a reaction inhibitor is effective for prolonging a reaction which relies upon the quantity of HDL cholesterol.

### Industrial Applicability

According to the present invention defined by claims 1 and 2, the cholesterol in HDL can be efficiently quantitated by simple procedures without needing pretreatment such as centrifugal separation. As a specific measurement is feasible with a sample in a small quantity by simple procedures, the present invention can be applied to various automatic analyzers and are extremely useful in the field of clinical tests.

## Claims

1. A method for the quantitation of cholesterol in high-density lipoprotein, which comprises the steps of adding to serum sample containing cholesterol in high-density lipoprotein:
i) a first reagent in the form of an aqueous solution containing 0.2 % polyoxyethylene-polyoxypropylene condensate ('Pluronic F-88'), 0.2% sodium phosphotungstate and 100mM magnesium chloride at pH 6.4: and
ii) a second reagent composed of 1% 'Emulgen A-60' which is a polyoxyethylene alkylene phenyl ether, 1U/ml cholesterol esterase. 1U/ml cholesterol oxidase, and 100mM MES buffer (pH 6.5) containing 0.04% disulfobutylmetatoluidine and 0.004% 4-aminoantipyrine, and then
iii) measuring the absorbance of the serum between 12 seconds and 312 seconds after the addition of the second reagent in order to determine from the difference in the absorbance measured the concentration of cholesterol in high-density lipoprotein by a 2-point method.

2. Use of a cholesterol in high-density lipoprotein quantitating reagent or reagent kit in the method of claim 1 wherein the reagent or reagent kit comprises the following ingredients (I) and (ii):
(i) a first reagent in the form of an aqueous solution containing 0.2 % polyoxyethylene-polyoxypropylene condensate ('Pluronic F-88', 0.2% sodium phosphotungstate and 100mM magnesium chloride at pH 6.4; and
(ii) a second reagent composed of 1% 'Emulgen A-60' which is a polyoxyethylene alkylene phenyl ether, 1U/ml cholesterol esterase, 1U/ml cholesterol oxidase, and 100mM MES buffer (pH 6.5) containing 0.04% disulfobutylmetatoluidine and 0.004% 4-aminoantipyrine.

## Patentansprüche

1. Verfahren zur quantitativen Bestimmung von Cholesterin in Lipoprotein mit hoher Dichte, mit den folgenden Schritten: Zugabe der folgenden Reagenzien zu einer Scrumprobe, die Cholesterin in Lipoprotein mit hoher Dichte enthält:
i) ein, erstes Reagens in Form einer wäßrigen Lösung, die 0,2% Polyoxyethylen-Polyoxypropylen-Kondensat ('Pluronic F-88'), 0,2% Natriumphosphorwolframat und 100 mmol Magnesiumchlorid bei einem pH-Wert von 6,4 enthält; und
ii) ein zweites Reagens, bestehend aus 1% '"Emulgen A-60', d. h. einem Polyoxyethylenalkylenphenylether, 1 Einhicit/ml Cholesterinesterase, eine Einheit/ml Cholesterinoxidase und 100 mmol MES-Puffer (pH 6,5), der 0,04% Disulfobutylmetatoluidin und 0,004% 4-Aminoantipyrin enthält, und dann
iii) Messen der Extinktion des Serums zwischen 12 Sekunden und 312 Sekunden nach der Zugabe des zweiten Reagens, um aus der Differenz der gemessenen Extinktion die Konzentration von Cholesterin in Lipoprotein mit hoher Dichte durch ein Zweipunktverfahren zu bestimmen.

2. Verwendung eines Reagents oder Reagenskits zur quantitativen Bestimmung von Cholesterin in Lipoprotein mit hoher Dichte bei dem Verfahren nach Anspruch 1, wobei das Reagens oder der Reagenskit die folgenden Bestandteile (i) und (ii) aufweist:
(i) ein erstes Reagens in Form einer waßrigen Lösung, die 0,2% Polyoxymethylen-Polyoxypropylen-Kondensat ('Pluronic F-88'). 0,2% Natriumpliosphorwolframat und 100 mmol Magnesiumchlorid bei einem pH-Wert von 6,4 enthält: und
(ii) ein zweites Reagens, bestehend aus 1 % 'Emulgen A-60. d. h. einem Polyoxyethylenalkylenphenylether, 1 Einheit/ml Cholesterinesterase, eine Einheit/ml Cholesterinoxidase und 100 mmol MES-Puffer (pH 6,5), der 0,04% Disulfobutylmetatoluidin und 0,004% 4-Aminoantipyrin enthält.

## Revendications

1. Procédé pour la quantification de cholesterol dans une lipoprotéine de haute densité, qui comprend les étapes consistant à ajouter à un échantillon de sérum contenant du cholestérol dans une lipoprotéine de haute densité:
i) un premier réactif sous la forme d'une solution aqueuse contenant 0,2% de condensat polyoxyétliylène-polyoxypropylene ("Pluronic F-88"), 0,2% de phosphotungstate de sodium et 100mM de chlorure de magnésium. à pH 6,4; et
ii) un second réactif composé de 1% de "Emulgen A-60", qui est un éther phénylique de polyoxyéthylène alkylène, 1U/ml de cholestérol estérase, 1U/ml de cholestérol oxydase et 100mM de tampon MES (pH 6,5) contenant 0,04 % de disulfobutylmètatoluidine et 0.004% de 4-aminoantipyrinc, puis
iii) à mesurer l'absorbance du sérum entre 12 secondes et 312 secondes après l'addition du second réactif afin de déterminer, d'après la différence d'absorbance mesurée, la concentration de cholestérol dans la lipoprotéine de haute densité par un procédé à 2 points.

2. Utilisation d'un réactif ou d'une trousse de réactif de quantification de cholestérol dans une lipoprotéine de haute densité dans le procédé selon la revendication 1, dans laquelle le réactif ou la trousse de réactif comprend les ingrédients (i) et (ii) suivants:
(i) un premier réactif sous la forme d'une solution aqueuse contentant 0,2% de condensat polyoxyéthylène-polyoxypropylène ("Pluronic F-88"), 0,2% de phosphotungstate de sodium et 100mM de chlorure de magnésium à pH 6,4; et
ii) un second réactif composé de 1 % de ""Emulgen A-60", qui est un éther phénylique de polyoxyethylène alkylène, 1U/ml de cholestérol estérase, 1U/ml de cholestérol oxydase et 100mM de tampon MES (pH 6,5) contenant 0,04% de disulfobutylmétatoluidine et 0,004 % de 4-aminoantipyrine.
